**Europäisches Patentamt**

**European Patent Office**

**Office européen des brevets**

(11) Numéro de publication : **0 458 681 A1**

# (12) DEMANDE DE BREVET EUROPEEN

(21) Numéro de dépôt : **91401264.6**

(51) Int. Cl.⁵ : **A61F 9/00, H01S 3/10**

(22) Date de dépôt : **16.05.91**

(30) Priorité : **21.05.90 FR 9006298**

(43) Date de publication de la demande :
**27.11.91 Bulletin 91/48**

(84) Etats contractants désignés :
**AT BE CH DE DK ES GB GR IT LI LU NL SE**

(71) Demandeur : **ALCON PHARMACEUTICALS LIMITED**
**Sinserstrasse 47**
**CH-6330 Cham (CH)**

(72) Inventeur : **Dacquay, Bruno**
**3, Allée de la Croix de l'arbre**
**F-63170 Aubiere (FR)**
Inventeur : **Chaduc, Jean-Paul**
**6, rue Barnier**
**F-63000 Clermont-Ferrand (FR)**

(74) Mandataire : **Bonnetat, Christian**
**CABINET BONNETAT 23, Rue de Léningrad**
**F-75008 Paris (FR)**

(54) **Dispositif à laser notamment destiné à des applications thérapeutiques.**

(57) La présente invention concerne un dispositif à laser destiné à agir sur une cible, comportant :

— un laser de puissance (3) comprenant une cavité résonante (4), ledit laser de puissance étant susceptible d'émettre un rayonnement de longueur d'onde spécifique, dirigé sur ladite cible par l'intermédiaire d'un dispositif optique (9),

— un laser de visée (15) susceptible d'émettre un rayonnement visible, dirigé sur ladite cible par l'intermédiaire dudit dispositif optique (9), le rayonnement émis par ledit laser de puissance étant aligné, en sortie dudit dispositif optique, avec le rayonnement émis par ledit laser de visée, et

— des moyens multiplicateurs de fréquence (11,12,14) susceptibles de modifier la longueur d'onde du rayonnement émis par le laser de puissance.

Selon l'invention, lesdits moyens multiplicateurs de fréquence (11,12,14) sont disposés dans ladite cavité résonante (4) du laser de puissance (3).

EP 0 458 681 A1

La présente invention concerne un dispositif à laser destiné plus particulièrement, quoique non exclusivement, à des applications thérapeutiques, telles que notamment le traitement des affections des yeux.

D'autres applications, par exemple industrielles et scientifiques, pourraient également être envisagées.

On sait que, notamment en ophtalmologie, les praticiens ont souvent besoin de disposer, selon la nature du traitement, de divers rayonnements de puissance émis par des lasers et caractérisés par leur longueur d'onde. A chaque type de laser utilisé (laser à argon, laser Nd-YAG, laser à colorant,...), correspond généralement un rayonnement de puissance dont la longueur d'onde est appropriée à un traitement spécifique.

Par exemple, pour effectuer une opération, telle qu'une iridectomie, sur un patient, le praticien utilise un laser de puissance du type Nd-YAG et un laser de puissance du type argon. Ainsi, pour cette opération, le sectionnement des tissus iridiens est réalisé au moyen du laser Nd-YAG, tandis que la coagulation desdits tissus est obtenue au moyen du laser à argon.

Beaucoup d'autres types d'intervention, notamment dans le domaine médical, nécessitent également l'usage d'au moins deux appareils à laser de puissance différents, ou d'un appareil comportant deux lasers distincts.

Par conséquent, les solutions de ce type pour de telles interventions impliquent une réalisation complexe et une maintenance suivie desdits appareils à laser, ainsi qu'un coût global élevé. Par ailleurs, l'encombrement qui s'ensuit est important et nécessite de prévoir des locaux adaptés.

On connaît également, notamment par la demande de brevet EP-A-0 007 256, un appareil de chirurgie ophtalmologique à laser, comportant un laser de puissance susceptible d'émettre un rayonnement de longueur d'onde spécifique sur une cible, en l'occurrence un oeil d'un patient, par l'intermédiaire d'un dispositif optique, un laser de visée susceptible d'émettre un rayonnement visible, dirigé sur ladite cible par ledit dispositif optique, le rayonnement émis par le laser de puissance étant aligné avec le rayonnement émis par le laser de visée, et des moyens pour modifier la longueur d'onde du rayonnement émis par le laser de puissance. Dans ce cas, un cristal de KDP peut convertir la longueur d'onde d'émission du laser de puissance de 1064 nanomètres en un rayonnement de 532 nanomètres. Cependant, le cristal doubleur de fréquence est prévu, dans l'appareil décrit par le document EP-A-0 007 256, à l'extérieur de la cavité résonante du laser de puissance. Cette disposition entraîne une perte notable de puissance lorsque l'on passe d'un mode de fonctionnement à l'autre, c'est-à-dire de la longueur d'onde de 1064 nanomètres à celle de 532 nanomètres, en rendant difficile l'adaptation de la puissance du laser au travail spécifique à effectuer, notamment la rupture des tissus iridiens, d'une part, et la coagulation de ces derniers, d'autre part.

La présente invention a pour but d'éviter les inconvénients cités, et concerne un dispositif à laser permettant de produire un rayonnement pouvant avoir deux longueurs d'onde distinctes, chacune d'elles étant spécialement adaptée à un travail spécifique, sans que le passage d'un mode de fonctionnement à l'autre n'entraîne une perte notable de puissance.

A cet effet, le dispositif à laser destiné à agir sur une cible, telle que, par exemple, une zone d'un oeil d'un patient, du type comportant :

– un laser de puissance, comprenant une cavité résonante délimitée par deux miroirs disposés de part et d'autre, respectivement, d'une source de rayonnement laser comportant un milieu actif, ledit laser de puissance étant susceptible d'émettre un rayonnement de longueur d'onde spécifique, dirigé sur ladite cible par l'intermédiaire d'un dispositif optique,

– un laser de visée susceptible d'émettre un rayonnement visible, dirigé sur ladite cible par l'intermédiaire dudit dispositif optique, le rayonnement émis par ledit laser de puissance étant aligné, en sortie dudit dispositif optique, avec le rayonnement émis par ledit laser de visée, et

– des moyens multiplicateurs de fréquence susceptibles de modifier la longueur d'onde du rayonnement émis par le laser de puissance, est remarquable, selon l'invention, en ce que lesdits moyens multiplicateurs de fréquence sont disposés dans ladite cavité résonante du laser de puissance.

Ainsi, grâce à l'invention, on peut obtenir, à partir d'un seul laser de puissance, un rayonnement pouvant avoir deux longueurs d'onde distinctes, c'est-à-dire, soit une longueur d'onde correspondant à celle engendrée par le laser de puissance, soit une longueur d'onde modifiée par lesdits moyens multiplicateurs de fréquence qui, grâce à leur agencement dans la cavité résonante du laser de puissance, permettent au rayonnement de longueur d'onde modifiée de conserver une puissance appropriée.

Avantageusement, la source de rayonnement du laser de puissance est du type à milieu actif Nd-YAG, tandis que la source de rayonnement du laser de visée est du type à milieu actif hélium-néon.

En particulier, lesdits moyens multiplicateurs de fréquence peuvent comprendre au moins un cristal doubleur de fréquence, adapté pour fournir, soit un rayonnement de longueur d'onde de 532 nanomètres à partir d'un rayonnement de longueur d'onde de 1064 nanomètres, soit un rayonnement de longueur d'onde de 660 nanomètres à partir d'un rayonnement de longueur d'onde de 1320 nanomètres.

De préférence, le cristal doubleur de fréquence est agencé entre une lame séparatrice susceptible d'être disposée sur le trajet du rayonnement émis par la source de rayonnement du laser de puissance et un miroir de renvoi supplémentaire de la cavité résonante.

Ainsi, dans l'exemple relatif à l'iridectomie mentionné préalablement, le praticien peut effectuer le sectionnement des tissus iridiens à l'aide du rayonnement émis par le laser de puissance Nd-YAG et dont la longueur d'onde peut être, par exemple, de 1064 nanomètres, et la coagulation desdits tissus à l'aide du rayonnement modifié par lesdits moyens multiplicateurs de fréquence et dont la longueur d'onde devient, sous l'action du cristal doubleur de fréquence, de 532 nanomètres. Cette longueur d'onde correspond approximativement à celle d'un laser à argon, qui est d'environ 500 nanomètres, utilisé usuellement dans ce cas. On obtient ainsi un dispositif à laser particulièrement adapté à l'iridectomie.

Bien entendu, on peut prévoir, selon le type d'intervention à effectuer, des laser de puissance et multiplicateur de fréquence les mieux appropriés.

Selon une autre caractéristique de l'invention, le laser de puissance comporte des moyens permettant que son rayonnement soit émis par impulsions.

Par ailleurs, le dispositif optique peut comprendre une pluralité de miroirs de renvoi définissant un premier trajet optique, et au moins une fibre optique, définissant un second trajet optique.

En particulier, un miroir escamotable peut être disposé sur le trajet du rayonnement visible émis par le laser de visée, de façon à diriger ledit rayonnement vers ladite fibre optique, et un miroir peut être disposé sur le trajet du rayonnement issu de ladite lame séparatrice, sortant du laser de puissance, de façon à diriger ledit rayonnement vers ladite fibre optique.

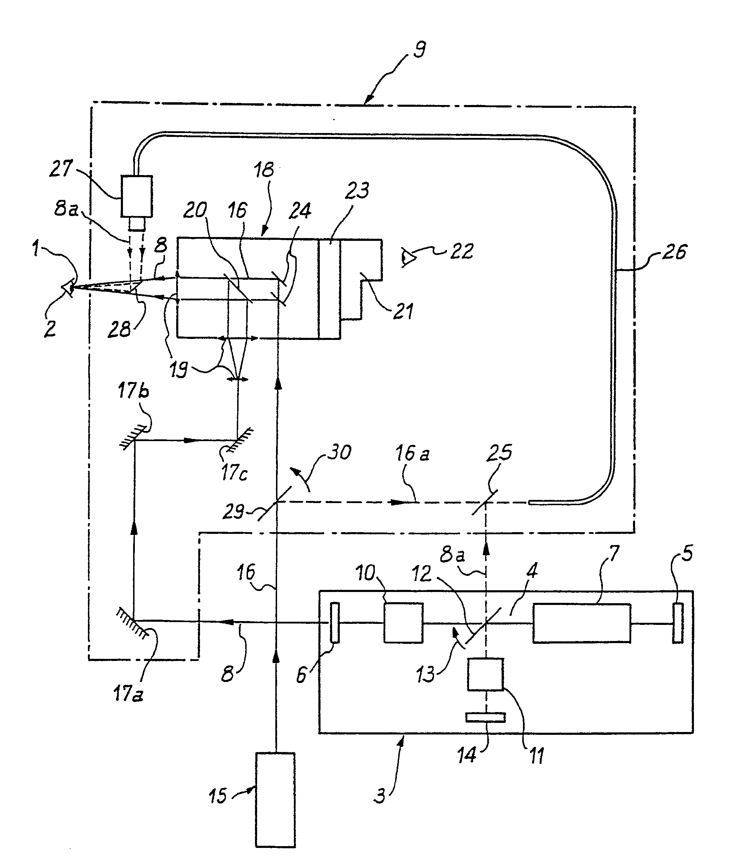

La figure unique du dessin annexé, représentant schématiquement un exemple de réalisation du dispositif à laser selon l'invention, fera bien comprendre comment l'invention peut être réalisée.

Dans cette application, le dispositif à laser selon l'invention est destiné à agir sur une zone 1 d'un oeil 2 d'un patient.

Le dispositif comporte un laser de puissance 3, comprenant une cavité résonante 4 délimitée par un premier miroir de renvoi 5 et un miroir semi-transparent 6, disposés de part et d'autre, respectivement, d'une source de rayonnement laser 7, comportant un milieu actif, par exemple Nd-YAG. Le laser de puissance 3 est susceptible d'émettre un rayonnement 8 de longueur d'onde spécifique, dirigé sur l'oeil 2 par l'intermédiaire d'un dispositif optique 9, décrit en détail par la suite. Par exemple, dans le cas de l'iridectomie, la longueur d'onde du rayonnement 8 issu du laser Nd-YAG 3 peut être de 1064 nanomètres, ce qui entraîne une rupture des tissus iridiens de la zone 1 de l'oeil 2 à traiter. Dans une autre application, la longueur d'onde du rayonnement 8 pourrait être de 1320 nanomètres, notamment. Un autre milieu actif peut être Nd-YLF.

Par ailleurs, le laser de puissance 3 peut comporter des moyens 10, tels qu'une cellule de Pockels ou un dispositif dit "Q-switch", permettant que son rayonnement soit émis par impulsions. Les moyens 10 peuvent être commutés pour fonctionner en continu ou quasi-continu, ou en impulsionnel. Pour obtenir un mode de fonctionnement quasi-continu, il suffit d'associer la source de rayonnement laser 7 à un élément du type acousto-optique (non représenté).

En outre, selon l'invention, on prévoit des moyens multiplicateurs de fréquence, susceptibles de modifier la longueur d'onde du rayonnement émis par le laser de puissance 3, disposés dans la cavité résonante 4 du laser de puissance 3. Ces moyens comprennent un cristal doubleur de fréquence 11, adapté pour fournir, notamment, des rayonnements de longueurs d'onde respectives de 532 et 660 nanomètres, selon le cristal particulier utilisé, à partir des rayonnements de longueurs d'onde de 1064 et 1320 nanomètres, respectivement, du laser Nd-YAG. On notera que la cavité résonante peut être optimisée sur 1320 nanomètres par translation des miroirs de cette dernière. Le cristal doubleur de fréquence 11 est agencé entre une lame séparatrice 12 susceptible d'être disposée sur le trajet du rayonnement émis par la source de rayonnement 7 du laser de puissance 3 (la lame 12 pouvant être escamotée par rotation de celle-ci comme indiqué par la flèche 13) et un second miroir de renvoi 14 de la cavité résonante 4. En particulier, dans le cas de l'iridectomie, le rayonnement de longueur d'onde de 532 nanomètres est utile pour procéder à la coagulation des tissus iridiens, cette longueur d'onde étant proche de celle émise par un laser du type argon. Le rayonnement dont la fréquence a été doublée sera dirigé vers la zone 1 de l'oeil 2 à traiter par l'intermédiaire du dispositif optique 9, en suivant un trajet optique avantageusement différent de celui suivi par le rayonnement "normal", comme on le verra en détail ci-après.

Il convient de préciser de plus que l'on peut utiliser un seul cristal dans la cavité résonante, pour obtenir une longueur d'onde de 532 nanomètres à partir d'une longueur d'onde de 1064 nanomètres, et une longueur d'onde de 660 nanomètres à partir d'une longueur d'onde de 1320 nanomètres et que, pour cela, il est nécessaire de faire pivoter le cristal pour que les axes soient en accord avec les longueurs d'onde souhaitées. On peut également utiliser deux cristaux que l'on commute sur le trajet optique.

Le dispositif à laser comporte de plus un laser de visée 15, par exemple du type hélium-néon, susceptible d'émettre un rayonnement visible 16, dirigé vers la zone 1 de l'oeil 2 à traiter par l'intermédiaire du dispositif optique 9. Il est bien entendu que le rayonnement émis par le laser de puissance 3 sera aligné, en sortie du dispositif optique 9, avec le rayonnement émis par le laser de visée 15.

Le dispositif optique 9 est adapté pour définir deux trajets optiques pour le rayonnement issu du laser de puissance, selon que celui-ci présente une fréquence "normale" ou doublée.

Le premier trajet optique, pour le rayonnement "normal" 8 qui peut être continu ou émis par impulsions, comporte une pluralité de miroirs de renvoi 17a,17b,17c, dirigeant le rayonnement 8 vers un appareil optique 18, schématiquement illustré, recevant et transmettant les rayonnements de puissance et de visée en direction de la zone 1 de l'oeil 2 du patient, à traiter. Cet appareil 18 comporte principalement un jeu de lentilles 19, un miroir réglable 20, et des moyens optiques de visée, tels qu'un microscope 21. On a représenté en 22 les yeux du praticien, observant à travers le microscope 21 l'oeil 1 du patient. Les yeux du praticien sont protégés par un filtre mobile ou fixe 23. Par ailleurs, des miroirs de renvoi 24 sont prévus dans l'appareil optique 18 pour renvoyer le rayonnement 16 émis par le laser de visée 15 en direction de la zone 1 de l'oeil 2 à traiter. Le rayonnement de puissance 8 est aligné sur le rayonnement de visée 16 au moyen du miroir réglable 20.

Le second trajet optique, pour le rayonnement 8a de fréquence doublée issu de la lame séparatrice 12, comporte un miroir 25 permettant de diriger ledit rayonnement vers une fibre optique 26, dans laquelle ce dernier se propage, pour aboutir à un zoom 27 au voisinage de l'appareil optique 18, un miroir 28 renvoyant le rayonnement 8a vers la zone 1 de l'oeil 2 à traiter. Par ailleurs, un miroir 29, escamotable par rotation selon la flèche 30, peut être disposé sur le trajet du rayonnement visible 16 émis par le laser de visée 15, de façon à diriger le rayonnement ainsi dévié 16a vers la fibre optique 26, cela lorsque les moyens multiplicateurs de fréquence sont actifs.

L'alignement des rayonnements de puissance 8 et 8a s'effectue à l'aide du laser de visée 15, ce qui permet de ne déclencher le laser de puissance 3 que pendant le traitement proprement dit, chaque rayonnement de puissance 8 et 8a correspondant, comme déjà indiqué, à un traitement spécifique (rupture des tissus iridiens ou coagulation de ceux-ci) de la zone 1 de l'oeil 2 à traiter.

Après réglage préalable du rayonnement 16 émis par le laser de visée 15, le praticien peut déclencher le tir du laser de puissance 3, dont le rayonnement, de longueur d'onde "normale" (par exemple de 1064 nanomètres), empruntera le premier trajet optique défini ci-dessus et sera utilisé pour rompre les tissus iridiens. Lorsque le praticien souhaite procéder à la coagulation desdits tissus, la lame séparatrice 12 est tournée pour qu'elle intercepte le rayonnement issu de la source 7. Le rayonnement est alors dévié vers le cristal doubleur de fréquence 11, puis renvoyé vers la fibre optique 26 en engendrant le rayonnement de puissance 8a dont la longueur d'onde sera, par exemple, de 532 nanomètres. Le rayonnement 16a, également dévié vers la fibre optique 26, du laser de visée permet l'alignement parfait du rayonnement de puissance 8a et du rayonnement de visée 16a.

Ainsi, grâce au dispositif de l'invention, on peut obtenir à partir d'une seule source de rayonnement laser, du type YAG ou YLF, et cela en continu, quasi-continu ou en impulsionnel, les différentes longueurs d'onde suivantes :

YAG 1064 nanomètres
   532 nanomètres
   1320 nanomètres
   660 nanomètres
YLF 1050 nanomètres
   525 nanomètres
   1310 nanomètres
   655 nanomètres

## Revendications

1 - Dispositif à laser destiné à agir sur une cible, telle que, par exemple, une zone d'un oeil d'un patient, du type comportant :

 – un laser de puissance, comprenant une cavité résonante délimitée par deux miroirs disposés de part et d'autre, respectivement, d'une source de rayonnement laser comportant un milieu actif, ledit laser de puissance étant susceptible d'émettre un rayonnement de longueur d'onde spécifique, dirigé sur ladite cible par l'intermédiaire d'un dispositif optique,

 – un laser de visée susceptible d'émettre un rayonnement visible, dirigé sur ladite cible par l'intermédiaire dudit dispositif optique, le rayonnement émis par ledit laser de puissance étant aligné, en sortie dudit dispositif optique, avec le rayonnement émis par ledit laser de visée, et

 – des moyens multiplicateurs de fréquence susceptibles de modifier la longueur d'onde du rayonnement émis par le laser de puissance, caractérisé en ce que lesdits moyens multiplicateurs de fréquence (11,12,14) sont disposés dans ladite cavité résonante (4) du laser de puissance (3).

2 - Dispositif selon la revendication 1, caractérisé en ce que la source de rayonnement (7) du laser de puissance (3) est du type à milieu actif Nd-YAG.

3 - Dispositif selon la revendication 1 ou la revendication 2, caractérisé en ce que la source de rayonnement du laser de visée (15) est du type à milieu actif hélium-néon.

4 - Dispositif selon l'une quelconque des revendications 1 à 3, caractérisé en ce que lesdits moyens multiplicateurs de fréquence comprennent au moins un cristal doubleur de fréquence (11).

**5** - Dispositif selon la revendication 4, caractérisé en ce que le cristal doubleur de fréquence (11) est adapté pour fournir un rayonnement de longueur d'onde de 532 nanomètres à partir d'un rayonnement de longueur d'onde de 1064 nanomètres.

**6** - Dispositif selon la revendication 4, caractérisé en ce que le cristal doubleur de fréquence (11) est adapté pour fournir un rayonnement de longueur d'onde de 660 nanomètres à partir d'un rayonnement de longueur d'onde de 1320 nanomètres.

**7** - Dispositif selon l'une quelconque des revendications 4 à 6, caractérisé en ce que le cristal doubleur de fréquence (11) est agencé entre une lame séparatrice (12) susceptible d'être disposée sur le trajet du rayonnement émis par la source de rayonnement (7) du laser de puissance (3) et un miroir de renvoi supplémentaire (14) de la cavité résonante (4).

**8** - Dispositif selon l'une quelconque des revendications précédentes, caractérisé en ce que le laser de puissance (3) comporte des moyens (10) permettant que son rayonnement soit émis par impulsions.

**9** - Dispositif selon l'une quelconque des revendications précédentes, caractérisé en ce que le dispositif optique (9) comprend une pluralité de miroirs de renvoi (17a-17c,20) définissant un premier trajet optique.

**10** - Dispositif selon l'une quelconque des revendications précédentes, caractérisé en ce que le dispositif optique (9) comprend au moins une fibre optique (26), définissant un second trajet optique.

**11** - Dispositif selon la revendication 10, caractérisé en ce qu'un miroir escamotable (29) peut être disposé sur le trajet du rayonnement visible émis par le laser de visée (15), de façon à diriger ledit rayonnement vers ladite fibre optique (26).

**12** - Dispositif selon la revendication 10 ou la revendication 11, caractérisé en ce qu'un miroir (25) peut être disposé sur le trajet du rayonnement issu de ladite lame séparatrice (12), sortant du laser de puissance (3), de façon à diriger ledit rayonnement vers ladite fibre optique (26).

Office européen
des brevets

**RAPPORT DE RECHERCHE EUROPEENNE**

Numero de la demande

EP 91 40 1264

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y,D | EP-A-0 007 256 (ARON-ROSA) <br> * Figure 1; page 6, lignes 14-20 * <br> --- | 1-9 | A 61 F 9/00 <br> H 01 S 3/10 |
| Y | US-A-4 841 528 (SIPES) <br> * Figure 1; colonne 3, ligne 61 - colonne 4, ligne 1 * <br> --- | 1-12 | |
| A | WO-A-8 500 010 (JAKO) <br> * Page 5, lignes 10-15; figure 1; page 4, lignes 3-7 * <br> --- | 10-12 | |
| A | US-A-4 852 115 (VIHERKOSKI) <br> * Colonne 2, lignes 52-56 * <br> --- | 1,6 | |
| A | US-A-3 769 963 (GOLDMAN) <br> * Colonne 8, lignes 64-67; figure 2 * <br> --- | 10-12 | |
| A | EP-A-0 297 360 (ALLIED-SIGNAL) <br> ----- | | |

**DOMAINES TECHNIQUES RECHERCHES (Int. Cl.5)**

A 61 B
A 61 F
H 01 S

Le présent rapport a été établi pour toutes les revendications

| Lieu de la recherche | Date d'achèvement de la recherche | Examinateur |
|---|---|---|
| LA HAYE | 13-07-1991 | BARTON S.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)